# EUROPÄISCHE PATENTSCHRIFT

(11) **EP 0 428 881 B1**
(45) Veröffentlichungstag und Bekanntmachung des Hinweises auf die Patenterteilung: **28.06.1995**
(21) Anmeldenummer: 90120271.3
(22) Anmeldetag: 23.10.1990
(51) Int. Cl.: C12N 9/00, C12N 15/11, C12N 5/10, A01H 5/00, A01N 57/16

(54) **RNA mit Endonuclease- und antisense-Aktivität, ihre Herstellung und ihre Verwendung**
RNA with endonuclease and antisense activity, its production and use
ARNs à activité endonucléase et antisens, leurs production et utilisation

(30) Priorität: 25.10.1989 DE 3935473
(43) Veröffentlichungstag der Anmeldung: 29.05.1991
(73) Patentinhaber: Hoechst Schering AgrEvo GmbH, 13342 Berlin (DE)
(72) Erfinder: Müllner, Hubert, Dr., W-6233 Kelkheim (Taunus) (DE); Schneider, Rudolf, Dr., W-6233 Kelkheim (Taunus) (DE); Uhlmann, Eugen, Dr., W-6246 Glashütten/Taunus (DE); Uijtewaal, Bernardus, Dr., NL-6093 JG Heythuysen (NL); Eckes, Peter, Dr., W-6233 Kelkheim (Taunus) (DE)

(56) Entgegenhaltungen:
- EP-A- 0 321 201
- PLANT MOLECULAR BIOLOGY, Band 11, 1988, S. 463-471, Kluwer Academic Publishers, Dordecht, NL; M.A. REZAIAN et al.
- BIOTECHNOLOGY, Band 6, Nr. 5, Mai 1988, S. 549-557, New York, US M. CUOZZO et al.

## Beschreibung

RNA-Moleküle können unter geeigneten Voraussetzungen ohne Beteiligung von Proteinen an anderen RNA-Molekülen Reaktionen katalysieren oder autokatalytisch Fragmente aus eigenen Molekülen abspalten. So wird vom 3′-Ende der 23S rRNA von Tetrahymena thermophila autokatalytisch ein Intron mit 413 Nukleotiden entfernt und in eine zirkuläre Form übergeführt. Dies geschieht durch eine Reihe von Phosphoestertransfer-Reaktionen mit Beteiligung von Guanosin-Kofaktoren (Cech, T.R., Nature 30, 578-583 (1983)). Je nach RNA Substrat oder den gewählten Reaktionsbedingungen kann das Intron als spezifische Ribonuclease, terminale Transferase, Phosphotransferase oder saure Phosphatase funktionieren. Dabei kann ein RNA-Molekül einige Umsetzungen vornehmen, ohne selbst verändert zu werden und verhält sich in dieser Einsicht wie ein Enzym. Für RNA-Moleküle mit diesen Eigenschaften wurde deshalb der Begriff Ribozym geprägt.

Ähnliche Reaktionen ohne Beteiligung von Proteinen konnten auch für einige viroide RNAs und Satelliten-RNAs gezeigt werden. So scheint für Avocado Sunblotch Viroid (ASBV) (Hutchins, C.J. et al. Nucleic Acids Res. 14, 3627-3640 (1986)), Satelliten-RNA von Tobacco Ringspot Virus (sTobRV) (Prody, G.A. et al., Science 231, 1577-1580 (1986)) und Satelliten-RNA von Luzerne Transient Streak Virus (sLTSV) (Forster A. C. et al., Cell 49, 211-220 (1987)) eine Selbst-Prozessierung eine essentielle Reaktion für die Vermehrung zu sein. Während der Replikation dieser RNAs werden vermutlich zirkuläre Formen gebildet, die als "Templates" zur Synthese von RNAs mit Überlänge führen. Diese Transkripte werden durch die selbstkatalysierten, endonucleolytischen Reaktionen auf die richtige Genomlänge zurechtgeschnitten.

Die Strukturen der RNAs, die diese vermutlich für die Reaktion einnehmen, wurden als "hammerheads" beschrieben (Forster A. C. et al., Cell 49, 211-220 (1987); Haseloff, J. et al., Nature 334, 585-591 (1988)).

Die Schnittstellen für diese RNA-Enzyme sind spezifisch, und müssen bestimmte strukturelle Voraussetzungen aufweisen, damit eine Prozessierung erfolgen kann.

Es wurde nun gefunden, daß Wirtszellen beliebiger Organismen mit Vektoren, die DNA kodierend für Ribozyme verknüpft mit "antisense"-RNA enthalten, transformiert werden können, so daß die genannte RNA exprimiert wird.

Es ist bekannt, daß "antisense"-RNA die Gen-Expression in einer Reihe von prokaryotischen und eukaryotischen Zellen, unter anderem auch in Pflanzenzellen inhibiert (Green, P., et al., Ann. Res. Biochem. 55, 569 (1986)). Der Hemmungsmechanismus ist noch weitgehend ungeklärt. Man nimmt an, daß sich in eukaryotischen Systemen doppelsträngige RNA bildet, die den Transport der m RNA zum Cytoplasma behindert.

Rezaian, M. et al., Plant Mol. Biol. 11, 463 (1988) untersuchten beispielsweise die Möglichkeit der Verwendung von "antisense"-RNA als antivirales Agens gegen Cucumber Mosaic Virus (CMV). Die Autoren beobachteten jedoch, daß die antivirale Aktivität der "antisense"-RNA unbefriedigend war.

Die Verknüpfung der entsprechenden Ribozym-RNA im "Loop" (Schema siehe unten) mit einer entsprechenden "antisense"-RNA resultiert nun jedoch in einer effektiveren Virusabwehr als Rezaian sie zeigen konnte. Eine derartige Verknüpfung der RNA-Moleküle bewirkt also nicht nur bezogen auf die Aktivität als antivirales Agens in Pflanzen, sondern allgemein eine verstärkte, gegen ein Substrat gerichtete Aktivität in transformierten Organismen.

Die Erfindung betrifft somit:
1. Ein Gen, kodierend für eine Ribozym-RNA-Sequenz im "Loop" verknüpft mit einer "antisense"-RNA-Sequenz.
2. Wirtszellen, die das unter 1. charakterisierte Gen enthalten.
3. Die Verwendung der durch das unter 1. charakterisierte Gen kodierten RNA als gegen ein Substrat gerichtetes Agens in Wirtszellen.

Im folgenden wird die Erfindung detailliert beschrieben, insbesondere in ihren bevorzugten Ausführungsformen. Ferner wird die Erfindung durch den Inhalt der Ansprüche bestimmt.

Die Ribozym/"antisense"-RNA kann gegen Substrate, wie z.B. RNA codierend für selektierbare Markergene (Antibiotika-Resistenzen) oder RNA codierend für beliebige Zellfunktionen, wie z.B. Dihydrofolatreduktase, Thymidinkinase, die Reifungsenzyme Polygalakturonase, Pektinesterase etc., Proteine verantwortlich für Differenzierung und Entwicklung oder Hormonrezeptoren, gerichtet sein. Insbesondere pflanzenschädigende Virusarten können vorteilhaft mit dem erfindungsgemäßen System bekämpft werden. Dazu wird beispielsweise wie im folgenden beschrieben vorgegangen. In analoger Weise kann auch eine Ribozym/"antisense"-RNA konstruiert werden, die gegen andere Substrate gerichtet ist.

Der Ribozym-Teil kann auf Basis der RNA-Sequenz des zu hemmenden Substrats synthetisiert werden. Bei einem Virus als Substrat kann es sich bei dieser RNA-Sequenz sowohl um das Genom von RNA-Viren handeln als auch um eine RNA-Sequenz, die von der DNA-Sequenz eines DNA-Virus abgeleitet wurde. Es kann also grundsätzlich von jedem pflanzenschädigenden Virus ausgegangen werden. Bevorzugte Virusarten sind pathogene Viren, insbesondere Cucumber Mosaic Virus, Brome Mosaic Virus, Alfalfa Mosaic Virus, Tabak Mosaic Virus, Kartoffel Virus X oder Y, Tomaten Ringspot Virus, Tomaten Aspermy Virus oder Tabak Rattle Virus. Insbesondere werden die RNA-Sequenzen RNA1, RNA2 und/oder RNA3 von Cucumber Mosaic Virus als Vorlage verwendet, entsprechend der Sequenz aus den Veröffentlichungen von Rezaian, M. et al. Eur. J. Biochem. 150, 331 (1985), Eur. J. Biochem. 143, 277 (1989) und Gould, J. et al. Eur. J. Biochem. 126, 217, (1982) oder jeweils Teile davon. Bevorzugt werden für die Synthese mindestens 10 aufeinanderfolgende Nukleotide, insbesondere 14 bis 20 Nukleotide, vorteilhaft aus der Mitte der RNA-Sequenz des entsprechenden Virus ausgewählt.

Die Ribozym kodierenden Oligonukleotide werden so synthetisiert, daß die jeweils aus mindestens 5 Nukleotiden, vorteilhaft 7 bis 10 Nukleotiden bestehenden Anfangs- bzw. Endsequenzen komplementär zu der RNA des zu hemmenden Virus sind. Die dazwischenliegende Sequenz besteht zum Teil aus spezifischen, für die Funktionalität des Ribozyms vorgegebenen Nukleotiden und zum Teil aus variablen Nukleotiden.

Bei der Herstellung der "antisense"-RNA wird entsprechend vorgegangen, nur werden die Oligonukleotide so synthetisiert, daß sie für eine RNA in entsprechender gegensinniger Orientierung kodieren.

Das Ribozym-Oligonukleotid wird im "Loop" mit einem entsprechenden Linker versehen. Derartige Linker besitzen beispielsweise Schnittstellen von EcoRI, SalI, BamHI, HindIII, EcoRV, SmaI, XhoI, KpnI, bevorzugt XbaI bzw. PstI. Über diesen Linker wird dann das synthetisierte Ribozym-Oligonukleotid mit dem für die "antisense"-RNA kodierenden Oligonukleotid im Ribozym-Loop miteinander verknüpft.

Das mit Substrat-RNA hybridisierte Ribozym/"antisense"-RNA System kann im Schema wie folgt aussehen: 
5-NNNNNNNNNNNNNGUC NNNNNNNNNNNNNNN-3 ← Substrat-RNA
3-KKKKKKKKCA KKKKKKKK-5
A C
U
A G
G A A
C G G V
V V
V V
VL VL
VL VL
VL VL
Ribozym
wobei
- N: Nukleotide der Substrat-RNA, A, C, G oder T,
- K: komplementäre Nukleotide zu N im Ribozym,
- V: variable Nukleotide im Ribozym und
- V_{L}: variable Nukleotide im "Loop" des Ribozyms bedeutet.
Die Nukleotidanzahl von V_{L} kann 0-550 betragen. V_{L}-Nukleotide werden so gewählt, daß auf Ebene der DNA eine Schnittstelle entsteht, in die Sequenzen eingefügt werden können.

Die zusammengesetzten Oligonukleotide werden mit Hilfe der Vektoren pUC19, pUC18 oder pBluescript (Stratagene, Heidelberg, Product Information) kloniert und sequenziert.

Das bestätigte Oligonukleotid wird in einen intermediären Vektor mit Pflanzenpromotor kloniert. Derartige Vektoren sind beispielsweise die Plasmide pPCV701 (Velten J. et al. EMBO J. 3, 2723-2730 (1984)), pNCN (Fromm H. et al. PNAS 82, 5824-5826 (1985)) oder pNOS (An, G. et al., EMBO J. 4, 277-276 (1985)). Bevorzugt wird der Vektor pDH51 (Pietrzak, M. et al., NAR 14, 5857, (1986)) mit einem 35S-Promotor verwendet.

Nach anschließender Transformation von E. coli, wie z.B. E. coli MC 1061, DH1, DK1, GM48 oder XL-1, werden positive Klone nach an sich bekannten Methoden (Maniatis et al., Lab. Manual), wie Plasmidminipräparation und Spaltung mit einem entsprechenden Restriktionsenzym, identifiziert.

Diese positiven Klone werden dann in einen binären Pflanzenvektor subkloniert. Als Pflanzenvektoren können pGV3850 (Zambrysk, P. et al, EMBO J. 2, 2143-2150 (1983)) oder pOCA18 (Olszewski, N., NAR 16, 10765-10782, (1988)) eingesetzt werden. Vorteilhaft wird mit pOCA18 gearbeitet.

Die erhaltenen binären Pflanzenvektoren, die einen Pflanzenpromotor mit dem angehängten DNA-Fragment für die Ribozym-Produktion in der T-DNA enthalten, werden verwendet, um Pflanzen zu transformieren. Dies kann durch Techniken wie Elektroporation oder Mikroinjektion erfolgen.

Bevorzugt wird die Kokultivierung von Protoplasten oder die Transformation von Blattstückchen mit Agrobakterien angewandt. Dazu wird das Pflanzenvektorkonstrukt durch Transformation mit gereinigter DNA oder, vermittelt über einen Helferstamm wie E. coli SM10 (Simon R. et al., Biotechnology 1, 784-791 (1983)) in Agrobakterium tumefaciens wie A282 mit einem Ti Plasmid über ein "triparental mating" transferiert. Direkte Transformation und "triparental mating" wurden, wie in "Plant Molecular Biology Manual" (Kluwer Academic Pubishers, Dordrecht (1988)) beschrieben, durchgeführt.

Es können grundsätzlich alle Pflanzen mit den die erfindungsgemäß konstruierte DNA tragenden binären Pflanzenvektoren transformiert werden. Bevorzugt sind dikotyledone Pflanzen, insbesondere Nutzpflanzen, die Stärke, andere Kohlenhydrate, Eiweiße oder Fette in nutzbaren Mengen in ihren Organen produzieren oder speichern oder die Obst und Gemüse produzieren oder die Gewürze, Fasern und technisch verwertbare Produkte oder Arzneimittel, Farbstoffe oder Wachse liefern und ebenfalls Futterpflanzen. Als Beispiel sollen Tomate, Erdbeere, Avocado, sowie Pflanzen, die tropische Früchte tragen, z.B. Papaya, Mango, aber auch Birne, Apfel, Nektarine, Aprikose oder Pfirsich genannt werden. Ferner sollen als zu transformierende Pflanzen beispielhaft alle Getreidearten, Raps, Rüpsen, Kartoffeln, Sojabohne, Baumwolle, Mais, Zuckerrübe oder Sonnenblume angeführt werden. Die transformierten Zellen werden mit Hilfe eines Selektionsmediums selektiert, zu einem Kallus herangezüchtet und auf einem entsprechenden Medium zur Pflanze regeniert (Shain et al., Theor. appl. Genet. 72, 770-770 (1986); Masson, J. et al., Plant Science 53, 167-176 (1987), Zhan et al., Plant Mol. Biol. 11, 551-559 (1988); McGranaham et al, Bio/Technology 6, 800-804 (1988) Novrate et al., Bio/Technology 7, 154-159 (1989).

Die resultierende Pflanze wird durch die Transformation insofern verändert, als in den Zellen mit Hilfe der konstruierten Oligonukleotide die entsprechende RNA exprimiert wird, wobei Ribozym RNA zusammen mit "antisense"-RNA gegen Virus-RNA aktiv werden kann.

Die folgenden Beispiele dienen der weiteren Erläuterung der Erfindung.

### Beispiele

Prozentangaben beziehen sich auf das Gewicht, soweit nichts anderes angegeben ist.

### 1. Synthese der DNA zur Expression von RNA

Die Synthese des Nukleotids für das Ribozym erfolgte auf Basis der CMV RNA1 Sequenz, Position 3248 bis 3264, (Rezaian M. et al., Eur. J. Biochem 150, 331-339 (1985)) versehen mit einem XbaI-Linker am 5′-Ende und einem PstI-Linker am 3′-Ende. Die konstanten Regionen ergeben sich aus dem beigefügten Schema für das Ribozym:

DNA zur Expression eines Ribozyms mit einer SalI-Stelle im variablen Teil des Loop: 
5'-CTAGAGGTAGCTCCTGATGAGTCGTCGACGACGAAACAACCTTCTGCA-3'
      TCCATCGAGGACTACTCAGCAGCTGCTGCTTTGTTGGAAG

oder:
5'-CTAGATTTCAAGGGTAGCTCCTGATGAGTCGTC
3'      TAAAGTTCCCATGGAGGACTACTCAGGAG

   GACGACGAAACAACCTTGTAGGATGTCTGCA-3'
   CTGCTGCTTTGTTGGAACATCCTACAG     -5'
Die Synthese des Nukleotids für die "antisense"-RNA erfolgte auf Basis der CMV RNA4 Sequenz (Gould I. et al., Eur. J. Biochem. 126, 217-26 (1982)) von Position 2179 bis 2193 mit einem XbaI-Linker am 5′-Ende und mit einem PstI-Linker am 3′-Ende, die zum Klonieren in die SalI-Stelle aufgefüllt wurden.

DNA zur Produktion einer "antisense"-RNA:

5'-CTAGATGGTCTCCTTATGGAGAACCTGTGGAAAACCACAGCTGCA-3'
TACCAGAGGAATACCTCTTGGACACCTTTTGGTGTCG

oder:
5'-TCGACATGGTCTCCTTATGGAGAACCTGTGGAAAACCACAGCG-3'
AG CT GTACCAGAGGAATACCTCTTGGACACCTTTTGGTGTCGCAGCT-5'

### 2. Klonierung in pBluescript SK+ und Sequenzierung

Das Plasmid pBluescript SK+ (Stratagene, Product Information) wurde mit den jeweiligen Enzymen geöffnet und nach Behandlung mit "calf intestinal phosphatase" (CIP) mit einem fünffachen Überschuß der doppelsträngigen phosphorylierten Oligonukleotide ligiert. Positive Klone konnten in dem Stamm XL-Blue (Stratagene) nach Induktion mit Isopropylgalactosid (IPTG) auf Platten mit 5-Brom-4-chlor-3-indolyl-β-D-galaktosid (X-gal) als weiße Kolonien identifiziert werden.

Jeweils 5 der Kolonien wurden isoliert und nach der Dideoxymethode sequenziert (Boehringer, Sequencing Kit), um die Orientierung des eingefügten Oligonukleotids zu bestimmen.

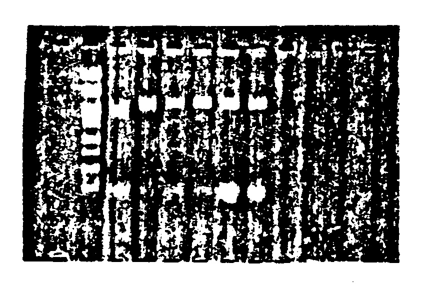

### Abb. 1:

pBluescript SK-Klone mit der gewünschten Oligonukleotid-DNA nach Xba/Pst Verdau.

### 3. Klonierung in pDH51

Das Plasmid pDH51 ist in der Veröffentlichung Pietrzak, M. et al. NAR 14, 5857-5868, 1986 nacharbeitbar beschrieben.

Das das Oligonukleotid enthaltende Plasmid pBluescript SK+ wurde mit XbaI und PstI verdaut, um nach der Fragmentauftrennung im 1 %igen "Low Melt"-Agarosegel die Oligonukleotid-DNA isolieren zu können.

Das isolierte Oligonukleotid wurde in fünffachem Überschuß in die Xba/Pst-Stelle von pDH51 eingebaut. Positive Klone konnten nach der Transformation von E. coli MC 1061 Zellen und Übertragen der Kolonien auf Nitrocellulosefilter durch Hybridisierung mit ³²P-markierter Oligonukleotid-DNA und anschließendem Waschen der Filter bei 65°C in SSC (1. 1 x SSC; 2. 0,1 x SSC; 3. 0,1 x SSC je 30 Min.) nachgewiesen werden.

In einen so erzeugten Vektor wurde über die neu geschaffene SalI-Schnittstelle eine DNA Sequenz zur Synthese der "antisense"- Oligonukleotid-DNA kloniert.

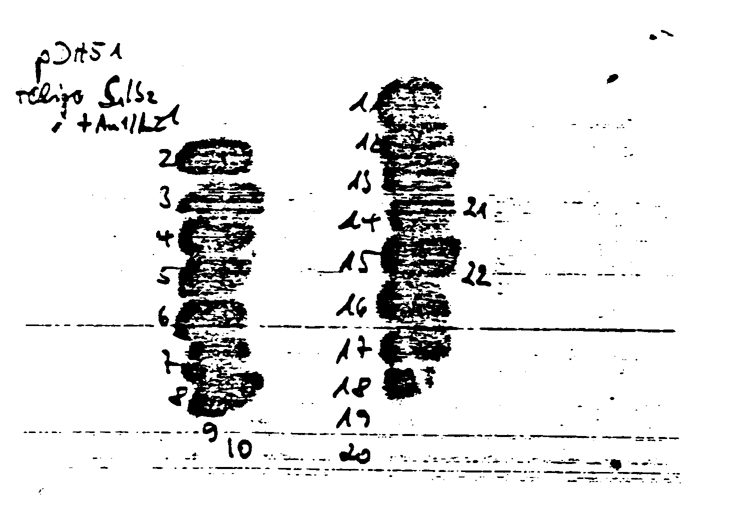

### Abb. 2:

Die Kolonien wurden nach Transformation von E. coli MC 1061 Zellen auf Nitrocellulosefilter ausgestrichen und über Nacht mit ³²P-markierter Oligonukleotid-DNA hybridisiert, die aus einem entsprechendem pBluescript SK+ isoliert wurde. Die Filter wurde wie oben genannt gewaschen. Die Autoradiographie erfolgte über Nacht.

### 4. Klonierung in pOCA18

Das Plasmid pOCA18 ist in der Veröffentlichung Olszewski, N. et al. NAR 16, 10765-10782, 1988, nacharbeitbar beschrieben. Das etwa 1,0 kb EcoRI Fragment aus pDH51 mit dem Oligonukleotid hinter dem 35S Promotor wurde über einen CsCl Gradienten isoliert und das durch EcoRI Verdau entstandene 0,8 kb Fragment in die EcoRI Schnittstelle von pOCA18 eingebaut.

Die positiven Klone wurden mittels einer DNA-Präparation und anschließendem EcoRI Schritt identifiziert.

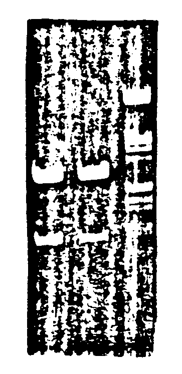

### Abb. 3:

- Reihe 1 + 2:: EcoRI geschnittene Klone nach der CsCl-Reinigung
- Reihe 3:: EcoRI/HindIII geschnittene λ-DNA

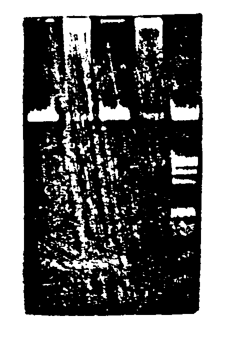

### Abb. 4:

- Reihe 1 + 3:: EcoRI geschnittene DNA aus der Ligasereaktion des Vektors pOCA18 mit dem 0,8 kb Fragment
- Reihe 2 + 4:: wie oben, jedoch ungeschnitten

### 5. Transformation von Agrobakterien

Der pOCA18 Vektor mit dem 35S Promotor/Oligonukleotid-Insert wurde in den Agrobakterienstamm A282 (Pharmacia, Freiburg, BR Deutschland oder ATCC 37349 USA) überführt. Dies geschah durch ein "Triparental Mating" mit Hilfe des E. coli Stammes SM10 (Simon, R. et al. Bio/Technology 1, 784-791, 1983). Dazu gab man gleiche Mengen der Bakterien gemeinsam über Nacht auf einen Filter, inkubierte diese bei 28°C, spülte mit 2 ml 10 mM MgSO₄ ab und gab Aliquots davon auf Hefeextrakt mit Tetrazyklin und Rifampicin (YEB: 1 % Yeast Extract, 1 % Pepton, 0,5 % NaCl), um positive Klone zu selektionieren. Die anschließende Hybridisierung der auf Nitrozellulosemembranen überführten Kolonien mit radioaktiv markierter Oligonukleotid-DNA bewies nochmals das Vorhandensein positiver Klone.

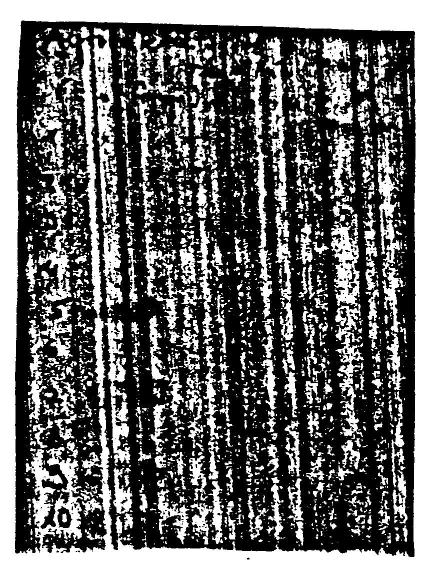
70

### Abb. 5:

Transformierte Agrobakterien wurden auf Filter ausgestrichen, über Nacht bei 28°C inkubiert und mit radioaktiv markierter Oligonukleotid-DNA hybridisiert.

### 6. Transformation von Tabak

Die Agrobakterien wurden in YEB Medium mit Tetrazyklin und Rifampicin angezüchtet. 20 ml der Bakterien wurden abzentrifugiert, einmal in YEB Medium gewaschen und in 20 ml 10 mM MgSO₄ suspendiert in eine Petrischale gegeben. Als Pflanzenmaterial wurde Nicotiana tabacum Wisconsin 38 verwendet. Die Pflanzen waren 4 Wochen unter sterilen Bedingungen auf 2MS Medium (Murashige, T. et al. Physiol. Plant 15, 473-497 (1962)) bei 25°C mit 16 Std. Licht pro Tag kultiviert worden. Von diesen Pflanzen wurde ein 1 cm² Blattstückchen abgeschnitten, mit sterilem Schmirgelpapier verwundet und 30 sec in die Übernacht-Bakterienkultur eingetaucht.

Die Blattstückchen wurden 2 Tage bei 25°C auf MS Medium, wie oben für 2MS beschrieben, gehalten und anschließend mit flüssigem 2MS Medium gewaschen. Danach kamen die Blattstückchen auf MSC 10 Platten (MS-Medium mit 1,5 % Agar) mit Kanamycin. Nach 5-6 Wochen konnten regenerierte Pflanzen in größere Gefäße umgepflanzt werden, wo sie nach 2-3 Wochen Wurzeln bildeten.

Die Expression der gewünschten RNA in den transgenen Pflanzen wurde durch Übertragen der im Gel (1 %iger Agarosegel mit 2,2 M Formaldehyd) aufgetrennten Gesamt-RNA (10 »g) auf Nitrozellulosefilter und anschließender Hybridisierung mit radioaktiver Oligonukleotid-DNA nachgewiesen.

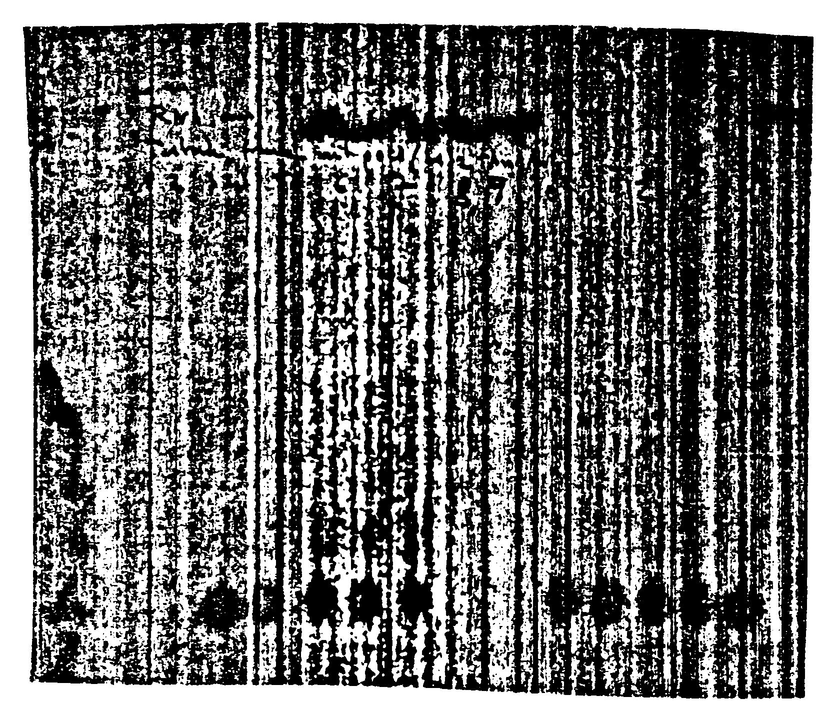

### Abb. 6:

- Reihe 1:: 10 »g Gesamt-RNA der Kontrollpflanze wurde nach Auftrennung im Gel auf Nitrozellulose übertragen. Die Hybridisierung erfolgte mit radioaktiv markierter Oligonukleotid-DNA
- Reihe 2-24:: wie vor, jedoch wurden jeweils 10 »g Gesamt-RNA der transgenen Pflanzen aufgetragen.

### 7. Nachweis der Transformation

Aus etwa 8 Wochen alten, transformierten Tabakpflanzen wurde mit Standardmethoden (Maniatis et al.; Lab. Journal) DNA isoliert, mit EcoRI geschnitten, jeweils 10 »g DNA auf Nitrozellulosemembranen transferiert und mit ³²P-markierter Oligonukleotid-DNA hybridisiert. In der DNA der transgenen Pflanze konnten durch Hybridisierung mit der charakteristischen EcoRI DNA der Einbau der gewünschten Sequenzen nachgewiesen werden.

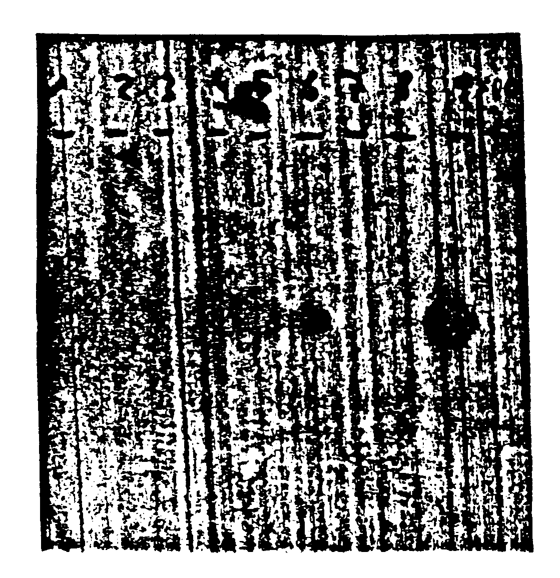

### Abb. 7:

- Reihe 1-9:: Je 10 »g Gesamt-DNA transgener Pflanzen wurden auf Nitrozellulose mit radioaktiv markierter Oligonukleotid-DNA hybridisiert.
- Reihe 10:: wie vor, jedoch Gesamt-DNA der Kontrollpflanze.

### 8. Nachweis der Expression der RNA

Aus den eben erwähnten Tabakpflanzen wurde aus einer zweiten Blattprobe RNA isoliert, aus einem Formaldehydgel auf Nitrozellulose transferiert und wie oben hybridisiert. Es konnten Banden nachgewiesen werden, die die erwarteten Größen zeigten.

### 9. Nachweis der in vitro Wirksamkeit der multifunktionellen RNA

Aus den pBluescript SK+ Klonen mit dem insertierten Gesamt-Oligo wurde durch Zugabe von T3 bzw. T7 Polymerase in einem Reaktionsansatz (Stratagene, Product Information zu SK+) RNA produziert und anschließend isoliert. Hybridisierung dieser RNA mit ³²P-markierter CMV RNA zeigte eine Aufspaltung der Virus-RNA.

### 10. Infektion mit CMV Stamm Q

Etwa 8 Wochen alte Tabakpflanzen wurden mit Hilfe von Carborundum mit dem CMV Stamm Q (Rezaian, M. et al., Eur. J. Biochem. 150, 331 (1985); 143, 277 (1984); Gould, J. et al., Eur. J. Biochem. 126, 217 (1982)) infiziert. Kontrollpflanzen zeigten nach 12-15 Tagen deutliche Symptome. In diesen Pflanzen konnten Viruspartikel nachgewiesen werden. Die transgenen Pflanzen erwiesen sich als unterschiedlich resistent gegen das Virus.

**Tabelle**

| Prozentsatz infizierter Pflanzen mit deutlichen Symptomen nach den jeweils angegebenen Zeiten (je 10 Pflanzen eines Klons wurden gemeinsam infiziert und ausgewertet) | | | |
|---|---|---|---|
| | Ausgangszahl der Pfl. | Anzahl der erkrankten Pfl. nach: | |
| | | 12 Tg. | 20 Tg |
| Wildtyp | 80 | 80 | 80 |
| transgene Pfl. | 3 x 10 | 40 | 50 |
| | 7 x 10 | 60 | 60 |
| | 10 x 10 | 40 | 60 |
| | 13 x 10 | 30 | 50 |
| | 18 x 10 | 60 | 60 |
| | 19 x 10 | 30 | 40 |
| | 20 x 10 | 30 | 30 |
| | 21 x 10 | 20 | 10 |
| | 22 x 10 | 50 | 50 |

## Patentansprüche (Patentansprüche für folgende(n) Vertragsstaat(en): AT, BE, CH, DE, DK, FR, GB, IT, LI, LU, NL, SE)

1. Gen kodierend für eine Ribozym-RNA-Sequenz "im Loop" verknüpft mit einer "antisense"-RNA-Sequenz.

2. Gen nach Anspruch 1, dadurch gekennzeichnet, daß die kodierte RNA zu einer Virus-RNA komplementär ist.

3. RNA kodiert durch ein Gen nach Anspruch 1 oder 2.

4. Wirtszelle, enthaltend ein Gen nach einem der Ansprüche 1 oder 2.

5. Wirtszelle, enthaltend eine RNA nach Anspruch 3.

6. Pflanzen, Pflanzenzellen sowie Teile oder Samen der Pflanzen, enthaltend das Gen nach Anspruch 1 oder 2.

7. Pflanzen, Pflanzenzellen sowie Teile oder Samen der Pflanzen, enthaltend die RNA nach Anspruch 3.

8. Verwendung der durch das Gen nach Anspruch 1 kodierten RNA als gegen ein Substrat gerichtetes Agens in pflanzlichen Wirtszellen.

9. Verwendung der durch das Gen nach Anspruch 2 kodierten RNA als gegen pflanzliche Viren gerichtetes Agens.

## Patentansprüche (Patentansprüche für folgende(n) Vertragsstaat(en): ES, GR)

1. Verfahren zur Synthese eines Gens kodierend für eine Ribozym-RNA-Sequenz, die im Loop mit einer "antisense"-RNA-Sequenz verknüpft ist, dadurch gekennzeichnet, daß
a) die Ribozym-kodierende Oligonukleotid-DNA auf Basis der RNA-Sequenz des zu hemmenden Substrats synthetisiert wird, so daß die jeweils aus mindestens 5 Nukleotiden, vorteilhaft 7 bis 10 Nukleotiden, bestehenden Anfangs- bzw. Endsequenzen komplementär zu der RNA des zu hemmenden Substrats sind und die dazwischenliegende Sequenz zum Teil aus spezifischen, für die Funktionalität des Ribozyms vorgegebenen Nukleotiden und zum Teil aus variablen Nukleotiden besteht,
b) die "antisense"-kodierende Oligonukleotid-DNA auf Basis des zu hemmenden Substrats so synthetisiert wird, daß sie für eine RNA in entsprechend gegensinniger Orientierung kodiert und
c) die synthetisierten Oligonukleotide über einen Linker im Ribozym-Loop miteinander verknüpft werden.

2. Verfahren nach Anspruch 1, dadurch gekennzeichnet, daß die Ribozym- sowie die "antisense"-kodierende Oligonukleotid-DNA so synthetisiert wird, daß sie komplementär kodierend zu einer viralen RNA ist.

3. Verfahren zur Transformation einer Wirtszelle, dadurch gekennzeichnet, daß
a) die nach Anspruch 1 oder 2 synthetisierte DNA mittels eines Vektors, der einen Promotor enthält, direkt oder mittels eines Helferstammes indirekt in die Zelle transferiert wird und
b) die transferierte DNA in der Wirtszelle exprimiert wird.

4. Verwendung der durch nach Anspruch 1 erhältlichen RNA als gegen ein Substrat gerichtetes Agens in Wirtszellen.

5. Verwendung der durch nach Anspruch 2 erhältlichen RNA als antivirales Agens.

## Claims (Claims for the following Contracting State(s): AT, BE, CH, DE, DK, FR, GB, IT, LI, LU, NL, SE)

1. A gene coding for a ribozyme RNA sequence in the loop coupled to an antisense RNA sequence.

2. The gene as claimed in claim 1, with the encoded RNA being complementary to a virus RNA.

3. An RNA encoded by a gene as claimed in claim 1 or 2.

4. A host cell containing a gene as claimed in one of claims 1 or 2.

5. A host cell containing an RNA as claimed in claim 3.

6. Plants, plant cells and parts or seeds of the plants, containing the gene as claimed in claim 1 or 2.

7. Plants, plant cells and parts or seeds of the plants, containing the RNA as claimed in claim 3.

8. The use of the RNA encoded by the gene as claimed in claim 1 as agent directed against a substrate, in plant host cells.

9. The use of the RNA encoded by the gene as claimed in claim 2 as agent directed against plant viruses.

## Claims (Claims for the following Contracting State(s): ES, GR)

1. A process for synthesizing a gene coding for a ribozyme RNA sequence which is coupled in the loop to an antisense RNA Sequence, which comprises
a) synthesizing the ribozyme-encoding oligonucleotide DNA on the basis of the RNA sequence of the substrate to be inhibited, so that the initial and final sequences, which are each composed of at least 5 nucleotides, advantageously 7 to 10 nucleotides, are complementary to the RNA of the substrate to be inhibited, and the intervening sequence is composed partly of specific nucleotides predetermined for the functionality of the ribozyme and partly of variable nucleotides,
b) synthesizing the antisense-encoding oligonucleotide DNA on the basis of the substrate to be inhibited, so that it codes for an RNA in appropriately anti-sense orientation, and
c) coupling the synthesized oligonucleotides together via a linker in the ribozyme loop.

2. The process as claimed in claim 1, wherein the ribozyme-and the antisense-encoding oligonucleotide DNA is synthesized so that it is complementarily coding to a viral RNA.

3. A process for transforming a host cell, which comprises
a) transferring the DNA synthesized as claimed in claim 1 or 2 by means of a vector which contains a promotor, directly or indirectly by means of a helper strain, into the cell, and
b) expressing the transferred DNA in the host cell.

4. The use of the RNA obtainable as claimed in claim 1 as agent directed against a substrate, in plant host cells.

5. The use of RNA obtainable through the gene as claimed in claim 2 as agent directed against plant viruses.

## Revendications (Revendications pour l'(les) Etat(s) contractant(s) suivant(s): AT, BE, CH, DE, DK, FR, GB, IT, LI, LU, NL, SE)

1. Gène codant pour une séquence d'ARN du ribozyme relié dans la boucle avec une séquence d'ARN "antisens".

2. Gène selon la revendication 1, caractérisé en ce que l'ARN codé est complémentaire à un ARN viral.

3. ARN codé par un gène selon la revendication 1 ou 2.

4. Cellule hôte contenant un gène selon une des revendications 1 ou 2.

5. Cellule hôte contenant un ARN selon la revendication 3.

6. Végétaux, cellules végétales ainsi que parties ou graines de végétaux contenant le gène selon la revendication 1 ou 2.

7. Végétaux, cellules végétales ainsi que parties ou graines de végétaux contenant l'ARN selon la revendication 3.

8. Utilisation de l'ARN codé par le gène selon la revendication 1, en tant qu'agent dirigé contre un substrat dans des cellules hôtes végétales.

9. Utilisation de l'ARN codé par le gène selon la revendication 2, en tant qu'agent dirigé contre des virus végétaux.

## Revendications (Revendications pour l'(les) Etat(s) contractant(s) suivant(s): ES, GR)

1. Procédé pour la synthèse d'un gène codant pour une séquence d'ARN ribozymique qui est lié dans la boucle à une séquence d'ARN "antisens" caractérisé en ce que
a) on synthétise l'ADN oligonucléotidique codant pour le ribozyme sur la base de la séquence d'ARN du substrat à inhiber, de sorte que chaque séquence initiale, respectivement terminale, constituée chaque fois d'au moins 5 nucléotides, avantageusement de 7 à 10 nucléotides, soient complémentaires à l'ARN du substrat à inhiber et que la séquence intercalée entre les deux se compose en partie de nucléotides spécifiques prèdéterminés pour la fonctionnalité du ribozyme, et en partie de nucléotides variables,
b) on synthétise l'ADN oligonucléotidique codant pour l'ARN "antisens" sur la base du substrat à inhiber de telle façon qu'il code pour un ARN orienté dans le sens opposé correspondant, et
c) on relie, l'un à l'autre, les oligonucléotides synthétisés par l'intermédiaire d'un linker dans la boucle du ribozyme.

2. Procédé selon la revendication 1, caractérisé en ce qu'on synthétise l'ADN oligonucléotidique codant pour le ribozyme ainsi que pour l'antisens de telle façon qu'il soit codant de façon complémentaire à un ARN viral.

3. Procédé pour la transformation d'une cellule hôte, caractérisé en ce que
a) on transfère l'ADN synthétisé selon la revendication 1 ou 2 à l'aide d'un vecteur qui contient un promoteur, directement ou indirectement par l'intermédiaire d'une souche auxiliaire dans la cellule, et
b) on exprime l'ADN transféré dans la cellule hôte.

4. Utilisation de l'ARN que l'on peut obtenir selon la revendication 1, en tant qu'agent dirigé contre un substrat dans le cellule hôte végétale.

5. Utilisation de l'ARN que l'on peut ontenir par l'intermédiaire du gène selon la revendication 2, en tant qu'agent dirigé contre les virus de végétaux.
